# EUROPEAN PATENT SPECIFICATION

(11) **EP 0 979 099 B1**
(45) Date of publication and mention of the grant of the patent: **15.06.2005**
(21) Application number: 98915471.1
(22) Date of filing: 10.04.1998
(51) Int. Cl.: A61K 38/40, A61P 37/08

(54) **USE OF LACTOFERIN IN THE TREATMENT OF ALLERGEN INDUCED DISORDERS**
VERWENDUNG VON LACTOFERRIN IN DER BEHANDLUNG VON BESCHWERDEN, DIE VON ALLERGENEN VERURSACHT SIND
UTILISATION DE LA LACTOFERRINE DANS LE TRAITEMENT DES TROUBLES INDUITS PAR LES ALLERGENES

(30) Priority: 10.04.1997 US 41890 P
(43) Date of publication of application: 16.02.2000
(73) Proprietor: AGENNIX, INC., Houston, TX 77054 (US)
(72) Inventor: KIMBER, Ian, Knutsford, Cheshire WA16 0DT (GB); CUMBERBATCH, Marie, Wilmslow, Cheshire SK9 6MN (GB); DEARMAN, Rebecca, J., Macclesfield, Cheshire SK10 1NG (GB); CONNEELY, Orla, M., Houston, TX 77030 (US); WARD, Pauline, Houston, TX 77030 (US)
(74) Representative: VOSSIUS & PARTNER
(86) International application number: PCT/US1998/007234
(87) International publication number: WO 1998/044940

(56) References cited:
- WO-A-93/13790
- WO-A-97/45136
- FR-A- 2 596 986
- FR-A- 2 641 696
- FR-A- 2 685 202
- DATABASE WPI Section Ch, Week 9544 Derwent Publications Ltd., London, GB; Class B04, AN 95-340208 XP002070022 & JP 07 233 086 A (MORINAGA MILK IND CO LTD)
- PATENT ABSTRACTS OF JAPAN vol. 097, no. 004, 30 April 1997 & JP 08 333260 A (KAMINOMOTO HONPO:KK), 17 December 1996,
- PATENT ABSTRACTS OF JAPAN vol. 096, no. 003, 29 March 1996 & JP 07 300425 A (SNOW BRAND MILK PROD CO LTD), 14 November 1995,
- PATENT ABSTRACTS OF JAPAN vol. 096, no. 007, 31 July 1996 & JP 08 059450 A (KOSE CORP), 5 March 1996,
- PATENT ABSTRACTS OF JAPAN vol. 018, no. 251 (C-1199), 13 May 1994 & JP 06 032743 A (MORINAGA MILK IND CO LTD), 8 February 1994,
- DATABASE WPI Section Ch, Week 9347 Derwent Publications Ltd., London, GB; Class B04, AN 93-374539 XP002070021 & JP 05 279 266 A (IMMUNO JAPAN KK)
- PATENT ABSTRACTS OF JAPAN vol. 097, no. 011, 28 November 1997 & JP 09 194388 A (MORINAGA MILK IND CO LTD), 29 July 1997,

## Description

### FIELD OF THE INVENTION

The present invention relates to pharmaceutical compositions and uses thereof for the treatment of an allergen incluced inflammatory disorder selected from asthma and contact dermatitis.

### BACKGROUND OF THE INVENTION

Lactoferrin (LF) is an 80 kilo Dalton (kD) iron-binding glycoprotein found in high concentrations in milk and in lower concentrations in other secretions and body fluids. It is one of a number of iron binding proteins, referred to as transferrins, involved in iron binding and delivery in mammals. Montreuil and Mullet, 1960, *C.R. Acad. Sci. Paris* 250:1736-1737; Montreuil *et al*., 1960, *Biochem. Biophys. Acta* 45:413-421; Johansson, 1960, *Acta. Chem. Scand.* 14:510-512, Blanc and Isliker, 1961, *Bull. Soc. Chim. Biol*. 43:929-943; Masson and Heremans, 1967, *Protides Biol. Fluids Proc. Colloq.* 21:115-124; Querinjenn *et al*., 1971, *Eur. J Biochem.* 20:420-425; Leger *et al*., 1977, *Biol. Anim. Biochim. Biophys.* 17:737-747.

Lactoferrin was originally discovered in milk where it can reach levels of 7 grams/liter in colostrum. Since then, however, it has been detected in a number of other body fluids including tears, saliva and mucosal secretions and also in the secondary granules of polymorphonuclear leukocytes. Biserte *et al*., 1963, *Exp. Ann. Biochim. Med*. 25:85-120; Masson, 1970, *in: La Lactoferrine*, pp. 93-165, Arscia, Bruxelles. Thus, the protein is expressed primarily by glandular epithelial cells and neutrophils associated with both local and central immune defense.

Lactoferrin has been shown to play important roles in host defense mechanisms due to its well-established antimicrobial activities. The antimicrobial actions of lactoferrin appear at least in part to be the consequence of the iron binding properties of lactoferrin *via* sequestration of iron necessary for microbial growth.

It has been shown that lactoferrin production is induced by lipopolysaccharides (LPS), which are components of bacterial cell walls. Gutteberg *et al*., 1990, *Scan. J. Clin. Lab. Invest*. 50:421-427. There is mounting evidence that in addition to antimicrobial activities, lactoferrin may influence innate and adaptive immune processes, including natural killer cell function, participate in the course of inflammation, and complement activation and affect cytokine production. Lash *et al*., 1983, *Blood* 61:885-888; Mansson *et al*., 1990, *Ann. Rheum. Dis.* 49:594-597; Van Snick *et al*.*,* 1974, *J. Exp. Med.* 140:1068-1084. With respect to the latter it has been demonstrated, both *in vivo* and *in vitro,* that lactoferrin may compromise the production of tumor necrosis factor α (TNF-α) systemically (Machnicki *et al*., 1993, *Int. J. Exp. Path.* 74:433-439), a cytokine that plays important roles in inflammation, sepsis and endotoxemic shock (Beutler *et al*., 1985, *Science* 229:869-871; Tracey *et al*., 1987, *Curr. Opinion Immunol*. 1:454-461; Waage *et al*., 1989, *J. Exp. Med*. 169:333-338; Kunkel *et al*., 1989, *Crit. Rev. Immunol*. 9:93-117).

In previous investigations, however, attention focused upon the regulation of TNF-α production provoked by LPS. Machnicki *et al*., 1993, *Int. J. Exp. Path.* 74:433-439; Gutteberg *et al*., 1990, *APMIS* 98: 1027-1032; and Gutteberg *et al*., 1991, *APMIS* 99:602-608. Since lactoferrin is known to bind directly to LPS, it was uncertain whether the observed reduction in TNF-α was attributable to transcriptional or post-transcriptional regulation of the cytokine itself or to the reduction by lactoferrin in the availability of LPS due to direct binding and inactivation of the endotoxin. Ellison and Giehl, 1991, J. *Clin. Invest*. 88:1080-1091; Appelmelk *et al*., 1994, *Infect. Immunity* 62:2628-2632. There has not been a previous demonstration of the allergen-induced effects of lactoferrin. For this reason, experiments have now been performed to examine the ability of homologous recombinant lactoferrin to influence the induction of TNF-α-dependent biological responses where stimulation of TNF-α expression is independent of LPS.

A cDNA encoding lactoferrin and methods for recombinantly producing the same have been disclosed by Conneely et al. in U.S. Patent Nos. 5,571,896, 5,571,697 and 5,57/1,691. Production of lactoferrin as a fusion product has been disclosed in U. S. Serial Nos. 08/453,703, 08/456,106 both filed May 30, 1995 and 08/691,123 filed on August 1, 1996. Moreover, the use of lactoferrin to modulate or neutralize heparin activity has been disclosed in U.S. Serial No. 08/391,986 filed on February 21, 1995. Additionally, lactoferrin mutants and variants thereof have been disclosed in U.S. Serial No. 08/866,544 filed on May 30, 1997. The disclosures of all the foregoing patents and applications are herein incorporated by reference in their entirety.

### SUMMARY OF THE INVENTION

Lactoferrin suppresses the induction of allergen dependent inflammatory responses. The present invention demonstrates that lactoferrin may inhibit TNF-α dependent responses that are not induced by an endotoxin, *i*.*e*., the lipopolysaccharide (LPS) component of the bacterial cell wall. Lactoferrin is an active compound for the treatment of a variety of inflammatory disorders that are the consequence of local immune reactions initiated by allergenic agents.

One aspect of the present invention features compositions suppressing local inflammatory reactions. The compositions comprises a lactoferrin and a pharmaceutically accepta,ble carrier. These compositions may alternatively, or in addition, include functional analogs or functional fragments of lactoferrin which exhibit the desired inhibitory activities on the locally induced TNF-α-dependent inflammation.

In another aspect, the invention provides methods for treating diseases characterized by a local immune reaction in a mammal by administering an effective amount of a composition comprising a lactoferrin and a pharmaceutically acceptable carrier. Compositions useful for the treatment of the present invention may alternatively, or in addition, include functional analogs or functional fragments of lactoferrin that exhibit inhibitory activity on the locally induced TNF-α dependent inflammatory reactions.

The compositions of the present invention are useful for treating a variety of indications that are the consequence of local immune reactions. Such indications include contact allergic dermatitis,

Finally, in another aspect, the compositions of the present invention may be employed for the treatment of asthma

### BRIEF DESCRIPTION OF THE DRAWINGS

FIGURE 1A depicts a cell migration cascade in the skin which results in a local immune response and inflammation.
FIGURE 1B depicts an interaction of cytokines produced by Langerhans cells and keratinocytes in the epidermis which triggers a local immune response in the skin.
FIGURE 2 depicts the binding of lactoferrin to its receptors on TNF-α producing keratinocytes *in situ* in neonatal mouse skin. The top two panels show the bright and the dark field images obtained using only labelled lactoferrin. The bottom two panels show the bright and the dark field images when the binding assay was performed in the presence of excess unlabelled lactoferrin.
FIGURE 3A demonstrates that intradermal administration of lactoferrin inhibits the accumulation of dendritic cells in draining lymph nodes induced by oxazolone, but not that stimulated by injection of TNF-α.
FIGURE 3B demonstrates that intradermal administration of lactoferrin inhibits both oxazolone and IL-1β-induced accumulation of dendritic cells in draining lymph nodes.
FIGURE 4 demonstrates the influence of lactoferrin on TNF-α and IL-1β-induced Langerhans cell migration.

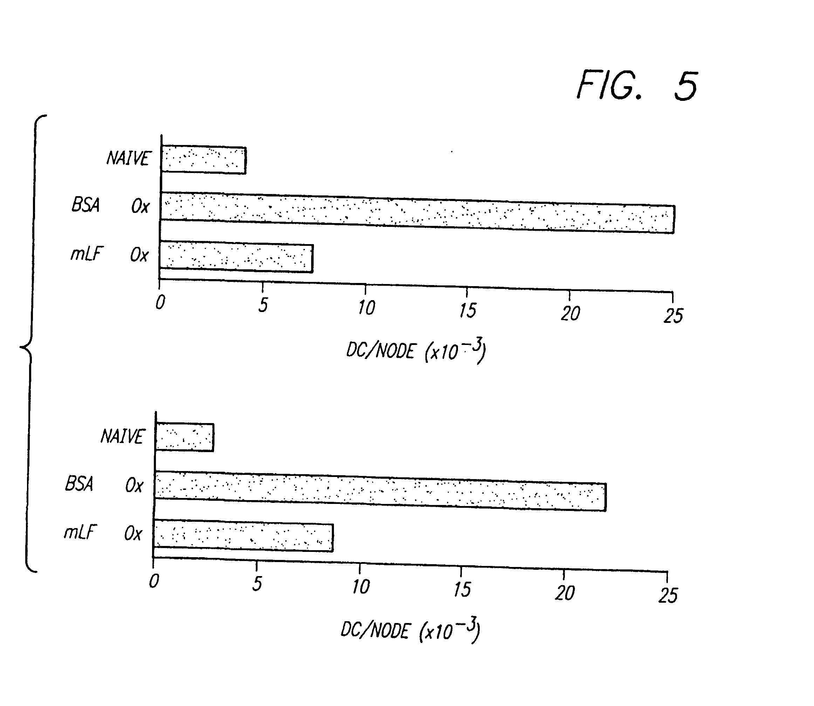

FIGURE 5 provides results from two independent experiments (panels A and B) indicate that treatment with oxazolone induced accumulation of dendritic cells in draining lymph nodes of mice pretreated with 0.02% BSA (see bar 2 panels A and B versus bar 1 control). Topical administration of mouse lactoferrin results in a strong inhibition of accumulation of dendritic cells in the lymph nodes in response to oxazolone in both experiments (see bar 3, panels A and B). The results indicate that topically administered lactoferrin is highly effective at inhibiting dendritic cell accumulation in lymph nodes in response to an allergen and hence allergen-induced cutaneous inflammation.

### DETAILED DESCRIPTION OF THE INVENTION

### Regulation Of Tumor Necrosis Factor α (TNF-α) Production By Lactoferrin

The present invention is based on the surprising discovery that lactoferrin and analogs and derivatives thereof including truncated molecules, biologically active fragments and muteins having substitutions and/or deletions are capable of inhibiting a variety of responses that require the production of TNF-α by endotoxin (LPS) independent pathways.

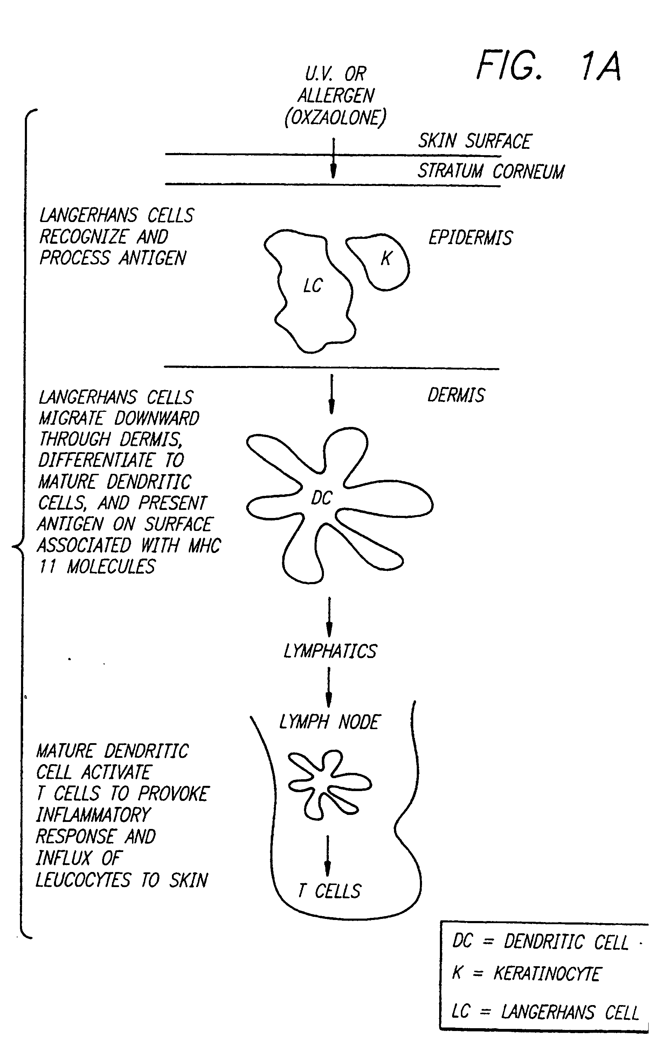

Inflammatory disorders are frequently the consequence of local immune reactions, e.g., caused by exposure to chemicals including exogenously and endogenously produced chemicals or allergens, or triggered by an autoimmune response. Such an inflammatory reaction is caused by trapping of the antigen by immune "Langerhans" cells that are localized in the epidermis. Once the antigen is trapped, the Langerhans cells undergo a maturation or differentiation process that allows them to dissociate from the epidermis and migrate as mature dendritic cells (DCs) downward through the dermis of the skin to the lymphatic system. They may then be carried *via* the lymphatic system to the draining lymph nodes where they present processed antigen on their cell surface to the T-lymphocytes. Kimber and Cumberbatch, 1992, *Toxicol. Appl. Pharmacol.* 117:137-146. *See,* FIGURE 1A. The activated T-cells may respond by triggering a complex immune reaction that results in invasion of the skin by inflammatory white blood cells to elicit a local inflammatory response.

Thus, local immune reactions, e.g., cutaneous immunity, requires:
(1) recognition and trapping of an antigen by Langerhans cell maturation and migration of dendritic cells to a lymph node;
(2) activation of T-cells in the lymph node by an antigen presenting dendritic cell; and
(3) subsequent stimulation of inflammatory white blood cell recruitment to the skin.

Each of these processes is dependent upon one or more cytokines that serve to regulate the function of the participating immune cells.

Cytokines that initiate an inflammatory response such as the cutaneous inflammatory response cascade are produced by both keratinocyte and Langerhans cells of the epidermis. Langerhans cells are localized in the epidermis in close apposition to keratinocyte cells as is evident from FIGURE 1B.

The induction of Langerhans cell migration by chemical allergens and the subsequent accumulation of dendritic cells in draining lymph nodes is dependent upon the local availability of TNF-α, a cytokine produced in the epidermis by keratinocytes in response to skin sensitization or other forms of dermal trauma. Cumberbatch and Kimber, 1992, *Immunology* 75:257-263; Cumberbatch *et al*., 1994, *Immunology* 81:395-401; Cumberbatch and Kimber, 1995, *Immunology* 84:31-35. Systemic administration of a neutralizing anti-TNF-α antibody almost completely inhibits the stimulation of Langerhans cell migration and dendritic cell accumulation induced by chemicals such as oxazolone. Cumberbatch and Kimber, 1995, *supra*. Intradermal administration of homologous recombinant TNF-α is itself sufficient to induce migration of Langerhans cells from the skin and to cause an increase in the number of dendritic cells found within draining lymph nodes. Cumberbatch and Kimber, 1992, *supra;* Cumberbatch *et al*., 1994, *supra*.

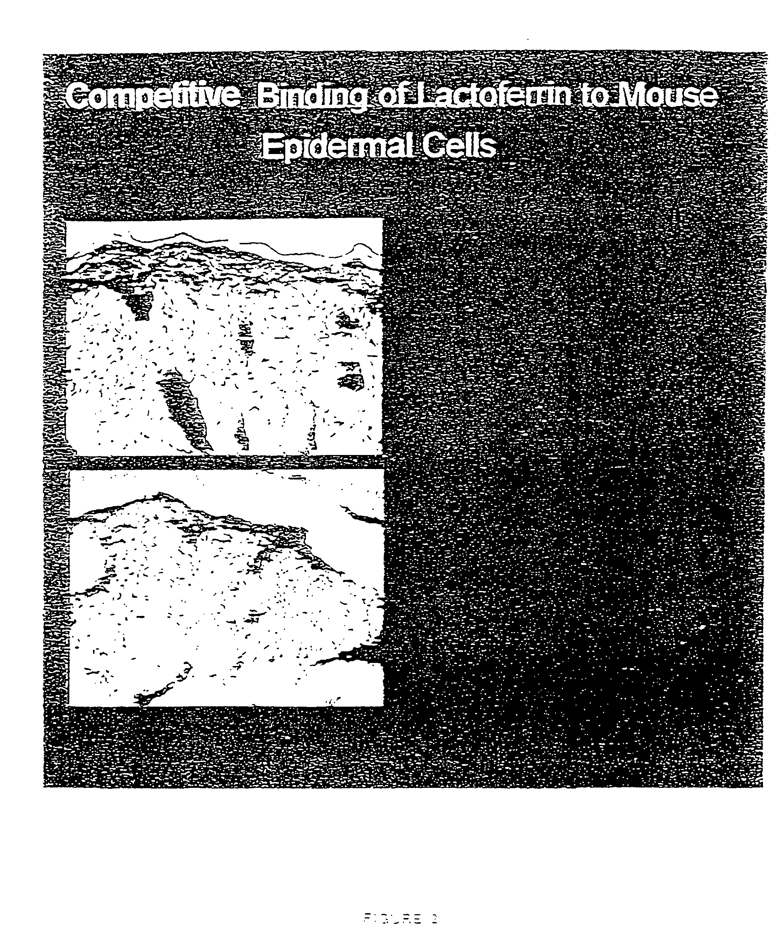

Intradermal administration of lactoferrin and functional analogs and functional fragments thereof inhibits the accumulation of dendritic cells in draining lymph nodes induced by oxazolone, but not that stimulated by injection of TNF-α as demonstrated by FIGURE 3A and Examples 2 and 3. These data are consistent with the negative regulation by lactoferrin of the production of TNF-α by keratinocytes that is known to be stimulated following skin sensitization.

It has previously been demonstrated that the stimulation of Langerhans cell migration and dendritic cell accumulation is dependent also upon the local availability of another epidermal cytokine, interleukin 1β (IL-1β). Enk *et al*., 1993, *J. Immunol*. 150:3698- 3704. This cytokine is produced constitutively in small amounts in murine epidermis by Langerhans cells and its expression is upregulated further as the result of skin sensitization. Enk and Katz, 1992, *Proc. Natl. Acad. Sci.* 89:1398; Enk and Katz, 1992, *J. Invest. Dermatol.* 99:S39-S41. Systemic administration of a neutralizing anti-IL-1β antibody inhibits Langerhans cell migration and dendritic cell accumulation. Enk *et al*., 1993, *supra*.

It is clear, therefore, that effective Langerhans cell migration is dependent upon the availability of IL-1β and the *de novo* synthesis by keratinocytes of TNF-α. This is further evidenced by previous work demonstrating that intradermal administration of IL-1β alone induces Langerhans cell migration. *See,* FIGURE 3B and Examples 2 and 3.

Lactoferrin inhibits both oxazolone-induced and IL-1β-induced dendritic cell accumulation in draining lymph nodes. Thus, lactoferrin impairs IL-1β-induced dendritic cell accumulation, but not that induced by TNF-α (*see,* FIGURE 3A). This demonstrates that the inhibitory effect of lactoferrin is downstream of IL-1β and upstream of TNF-α in the cytokine cascade triggering the local immune response.

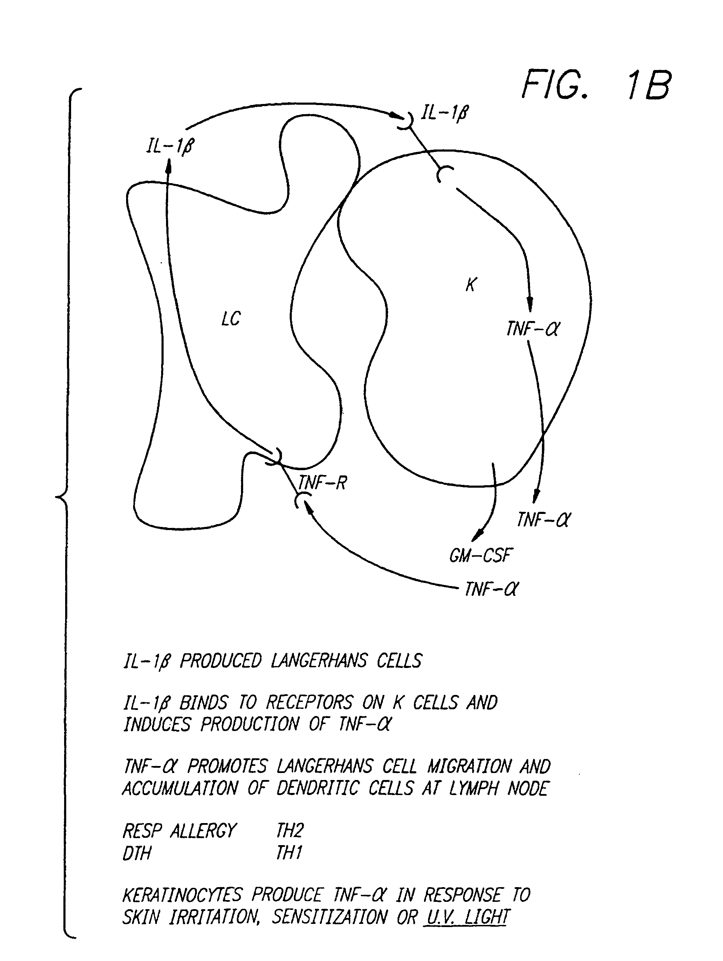

Further, it is demonstrated herein that lactoferrin binds to receptors on keratinocytes, indicating that binding of lactoferrin to keratinocytes prevents the production of TNF-α. Specifically, FIGURE 2 demonstrates the binding of lactoferrin to its receptors on TNF-α producing keratinocytes *in situ* on neonatal mouse skin. The binding of lactoferrin is concentrated in the epidermal layer and in the sweat glands, but not in the normal epidermal cells.

The experimental results disclosed herein demonstrate that the inhibitory effect of lactoferrin on Langerhans cell migration and dendritic cell accumulation is due to the negative regulation of TNF-α production, rather than *via* an influence on another aspect of Langerhans cell function. Thus, lactoferrin is able to inhibit the *de novo* synthesis of TNF-α by binding on receptors on keratinocytes.

The evolution of a two lobe structure has endowed lactoferrin with unique iron binding properties and contributed to other biological functions of the molecule. Based on identification of those domains, sequences and structures in the lactoferrin polypeptide contributing to the iron binding properties of the protein are described in U.S. Serial No. 08/866,544 filed May 30, 1997, the disclosure of which is herein incorporated by reference. This application provides guidance for the design and generation of novel lactoferrin variants or portions thereof having a modified iron binding capacity. Typically, lactoferrin variants have improved properties, including, but not limited to, lactoferrin variants with higher affinity for iron for improved antimicrobial activities, lactoferrin variants with lower affinity for iron having improved iron-releasing properties, or lactoferrin variants having modified pH or temperature requirements or ranges for the binding and/or release of iron. Likewise, lactoferrin variants useful in the methods of the present invention typically provide enhanced inhibition of interleukin activity such as that demonstrated by IL-1β. In addition, the invention allows for the design of lactoferrin variants having otherwise improved characteristics, e.g., therapeutic tolerance, immunoreactivity, or biological half life, while retaining their biological activity.

The lactoferrin variants of the invention may be derived from *wild-type* lactoferrin of a variety of mammalian species, including, but not limited to, human, murine, rate, bovine, and porcine lactoferrin. The *wild-type* lactoferrin may be mutated by a variety of methods generally known in the art. *See,* among other places, Sambrook *et al*., 1990 *Molecular Cloning; A Laboratory Manual*. Cold Spring Harbour Laboratory Press, New York; Kunkel *et al*., 1987, *Meth. Enzymol*. 154:367-382; Kunkel, 1985, *Proc. Natl. Acad. Sci*. USA 82:488-42.

In a preferred embodiment, the lactoferrin variants of the present invention comprise at lease one mutation in the amino acid sequence. In another preferred embodiment, the lactoferrin variants of the present invention comprise a truncated amino acid sequence.

The nucleic acid sequences encoding lactoferrin and variants thereof according to the present invention may be inserted in a vector suitable for expression in a eukaryotic cell in such way that allows expression of the lactoferrin variant. Alternatively, nucleic acid sequences encoding portions of the lactoferrin variants of the invention may be inserted in vectors allowing their expression in eukaryotic cells.

In another preferred embodiment, lactoferrin is produced in a recombinant expression system. *See,* e.g., Ward *et al*., 1992, Biotechnology 10:784-789; Ward *et al*., 1995, Biotechnology 13:498-503. For this purpose, nucleic acids coding for the desired form of lactoferrin (*see, e.g.,* U.S. Patent 5,571,691, incorporated by reference in its entity) is incorporated expressibly in a cellular host, which is then cultured under conditions appropriate for expression of that particular peptide or protein. A variety of gene expression systems have been adapted for this purpose, and typically drive expression of the desired gene from expression controls used naturally by the chosen host.

Because the lactoferrin variants of the invention typically, as natural occurring lactoferrin, requires post-transnational modifications, such as glycosylation at several amino acid residues, many of the lactoferrin variants or portions thereof need to be produced in an eukaryotic host. In preferred embodiments, the lactoferrin product is produced by an *Aspergillus* expression system, as described in Ward *et al*., 1992, *Gene* 122:219-223; and U.S. patent Nos. 5,571,896 and 5,571,697, the disclosures of which are herein incorporated by reference in their entirety.

If unglycosylated forms of lactoferrin variants or portions thereof are produced, however, their production may conveniently be achieved in bacterial hosts such as *E. Coli.* For such production, a nucleic acid coding for the selected lactoferrin variant or portion thereof, may usefully be placed under an expression control, *e*.*g*., of the lac, trp or PL genes of *E. coli.*

As an alternative to expression of nucleic acid coding for the lactoferrin variant or portion *thereof per se,* the host can be adapted to express the lactoferrin product as a fusion protein in which the lactoferrin product is linked releasably to a carrier protein that facilitates isolation and stability of the expression product.

In a further alternative, the lactoferrin variant or portion thereof may be generated by organic synthesis. In particular where production of a portion of alactoferrin variant, e.g., a peptide of about twenty (20) through about fifty (50) amino acids in length, is the objective preferably the well established techniques of automated peptide synthesis are employed, generally descriptions of which appear, for example, in J.M. Stewart and J.D. Young, Solid Phase Peptide Synthesis, 2nd Edition, 1984, Pierce Chemical Company, Rockford, Illinois; and in M. Bodanszky and A. Bodanszky, The Practice of Peptide Synthesis, 1984, Springer-Verlag, New York; Applied Biosystems 430A Users Manual, 1987, ABI Inc., Foster City, California; and Solid Phase Peptide Synthesis. A practical Approach, by: E. Atherton & R.C. Sheppard, IRL Press, Oxford (1989). In these techniques, the lactoferrin variant portion is grown from its C-terminal, resin-conjugated residue by the sequential addition of appropriately protected amino-acids, using either the Fmox or tBox protocols.

### Pharmaceutical Compositions Containing Lactoferrin

***Lactoferrin And Lactoferrin Products.*** The term "lactoferrin" refers collectively herein to naturally or recombinantly produced forms of lactoferrin, particularly mammalian forms unless otherwise specified. The term "lactoferrin products" as used herein refers collectively to lactoferrin and functional analogs and functional fragments thereof which may be useful to practice the invention. Specifically, the term encompasses, for example, truncated lactoferrin and lactoferrin having one or more amino acids substituted or deleted. "Functional analogs and functional fragments" of lactoferrin refer to lactoferrin mutants and derivatives, and lactoferrin derived peptides, respectively, which have the capacity to inhibit production of TNF-α in a cell. Generally, the lactoferrin, functional lactoferrin analogs and functional fragments thereof encompassed by the invention are capable of inhibiting an inappropriate or exaggerated local immune response that involves the production of TNF-α.

Lactoferrin analogs or lactoferrin derived peptides can be tested for identification of functional analogs or functional fragments by, for example, the mouse model described herein in Example 2. *See also*, Cumberbatch and Kimber, 1992, *Immunology* 75:257-263. Briefly, this testing involves induction of a local immune response by administering an allergen, e.g., oxazolone on the shaved skin of mice. One hour later, mice are injected intradermally at the oxazolone treated site with about 0.002% to about 0.5% lactoferrin analog or fragment to be tested in saline. A second and a third group of animals are injected with lactoferrin and BSA, respectively, at the same range of concentrations and in the same buffer. After about 12 hours, the animals may be killed, and the draining lymph nodes recovered. Comparison of the numbers of accumulated dendritic cells, which may be determined as described in Cumberbatch and Kimber, *supra*, will reveal the efficacy of a lactoferrin analog or fragment and its usefulness for the compositions and methods of the present invention.

Generally, any substitution, addition or deletion of lactoferrin that does not destroy its receptor mediated inhibitory effects on the production of TNF-α as part of an inappropriate or exaggerated immune response may be usefully employed in this invention. Those of ordinary skill in the art can make such substitutions, addditions and deletions without undue experimentation. In preferred embodiments, the functional lactoferrin analogs or functional fragments are substantially as effective as native human lactoferrin. In the most preferred embodiments, the functional lactoferrin analogs or functional fragments have enhanced TNF-α downregulating activity compared with native human lactoferrin. For example, such functional analogs or functional fragments may exhibit enhanced serum stability, enhanced receptor binding and enhanced signal transducing activity. Other modifications to lactoferrin and functional lactoferrin analogs and functional fragments that may usefully be employed in this invention are those which render the molecule more readily bioavailable to sites of inflammation. For example, in cases where the lactoferrin product is administered topically, the functional lactoferrin analog or functional fragment may exhibit enhanced percutaneous absorption.

The particular lactoferrin products according to the present invention may be prepared by a variety of techniques well known for generating protein products. Those forms of lactoferrin that occur naturally can of course be obtained by extraction from the natural source, e.g., mammalian, most preferably human, milk, using an appropriate combination of protein isolation techniques. For example, as described in Cheron *et al*., 1977, *C.R. Acd. Sci. Paris* 284:585-588, lactoferrin isolation is achieved by sequential extraction and purification with Sephadex G-25, G-50, G-75, and G-100.

As an alternative to extraction, those forms of lactoferrin that incorporate only L-amino acids may be produced reproducibly and in commercial quantities by application of recombinant DNA technology. *See, e.g.,* Ward *et al*., 1992, *Biotechnology* 10:784-789; Ward *et al*., 1995, *Biotechnology* 13:498-503. For this purpose, nucleic acids coding for the desired form of lactoferrin (*see, e.g.,* U.S. Patent 5,571,691, incorporated by reference in its entity) may be incorporated expressibly in a cellular host that is then cultured under conditions appropriate for expression of that particular peptide or protein. A variety of gene expression systems have been adapted for this purpose and typically drive expression of the desired gene from expression controls used naturally by the chosen host.

Because lactoferrin in its natural form requires post-translational modifications, such as glycosylation at several amino acid residues, many of the lactoferrins, functional analogs, or functional fragments thereof according to the present invention need to be produced in a eukaryotic host. In preferred embodiments, the lactoferrin product may be produced by an *Aspergillus* expression system, as described by Ward *et al*., 1992, *Gene* 122:219-223; and U.S. Patent Nos. 5,571,896 and 5,571,697, herein incorporated by reference in their entirety.

If unglycosylated forms, functional analogs, or functional fragments of lactoferrin are used, however, their production may conveniently be achieved in bacterial hosts such as *E. coli.* For such production, a nucleic acid encoding the selected lactoferrin, functional analog, or functional fragment thereof may be placed under an expression control of, *e.g.,* of the lac, trp or PL genes of *E. coli.*

As an alternative to expressing a nucleic acid encoding the lactoferrin, functional analog, or functional fragment thereof *per se,* a host may be adapted to express the lactoferrin product as a fusion protein in which the lactoferrin product is linked releasably to a carrier protein that facilitates isolation and stability of the expression product.

In a further alternative, the lactoferrin product may be generated by organic synthesis. In particular where the lactoferrin product is a functional fragment, e.g., a peptide of about twenty (20) through about fifty (50) amino acids in length, the well established techniques of automated peptide synthesis are employed. General descriptions of exemplary peptide syntheses appear for example, in J.M. Stewart and J.D. Young, Solid Phase Peptide Synthesis, 2nd Edition, 1984, Pierce Chemical Company, Rockford, Illinois; and in M. Bodanszky and A. Bodanszky, The Practice of Peptide Synthesis, 1984, Springer-Verlag, New York; Applied Biosystems 430A Users Manual, 1987, ABI Inc., Foster City, California; and Solid Phase Peptide Synthesis - A Practical Approach, by: E. Atherton & R.C. Sheppard, IRL Press, Oxford (1989). In these techniques, the lactoferrin fragment may be grown from its C-terminal, resin-conjugated residue by the sequential addition of appropriately protected amino acids, using either the Fmoc or tBoc protocols.

In an alternative approach, functional lactoferrin fragments useful for the methods and compositions of the present invention may be obtained by peptidase digest or hydrolysis of a purified lactoferrin or lactoferrin products. Of course, lactoferrin fragments may also be produced by recombinant expression in a suitable host as set forth, *supra*. The invention also encompasses the use of chemically modified lactoferrin products, including methylations, carboxylation, etc.

***Pharmaceutical Formulation And Routes Of Administration*****.** For therapeutic use, the lactoferrin product(s) according to the present invention may be administered by any conventional means available for use in conjunction with pharmaceuticals, either as individual therapeutic agents or in a combination of therapeutic agents. Each can be administered alone but is generally administered with a pharmaceutical carrier selected on the basis of the chosen route of administration and standard pharmaceutical practice. The pharmaceutical compositions of the invention may be adapted for oral, parenteral, topical or rectal administration, or as an inhalant, and may be in unit dosage form, in a manner well known to those skilled in the pharmaceutical art. Parenteral administration includes but is not limited to, injection subcutaneously, intravenously, intraperitoneally or intramuscularly.

Treatment with the active ingredient may begin at any time after the indication to be treated, *e.g*., psoriasis, contact dermatitis, UV-induced inflammation, infant diaper rash, asthma, arthritis, and the like, is diagnosed. Preferably, treatment is commenced as a prophylactic or at early stages of the disease, in order to prevent massive inflammation in the first place. Typically, treatment will continue until the inflammation is cured. In cases of chronic diseases, such as psoriasis, asthma or arthritis, or in cases of continued exposure to an allergen, the treatment may have to be extended beyond the cure of the symptoms. Because lactoferrin is a naturally occurring non-toxic protein, side effects are not expected even in cases of a long-term treatment.

The dose administered will, of course, vary depending upon known factors, such as (1) the pharmacodynamic characteristics of the particular lactoferrin product and its mode and route of administration, (2) the age, health, height and weight of the recipient, (3) the nature and extent of the symptoms, (4) the kind of concurrent treatment(s), (5) the frequency of treatment(s), and (6) the effect desired. A daily dose of active ingredient can be expected to be about 1 milligram to about 1.2 grams per kilogram of body weight, with the preferred dose being 50 milligrams to about 500 milligrams per kilogram of body weight. When the active ingredient is administered topically to the skin or in an inhalant, the active ingredient is typically applied in 0.005% to about 5% admixed with the carrier, preferably being about 0.05% to about 0.5% admixed with the carrier.

Dosage forms (compositions suitable for administration) contain from about 0.005 % to about 0.5 % of active ingredient per unit. In these pharmaceutical compositions, the active ingredient is ordinarily present in an amount of about 0.5-95% by weight based on the total weight of the composition.

Typically, the active ingredient will be administered topically, as inhalant, or as injection in inflamed joints or cartilage. However, alternatively the lactoferrin products may be administered orally in solid or semi-solid dosage forms, such as hard or softgelatin capsules, tablets, or powders, or in liquid dosage forms, such as elixirs, syrups, or suspensions. It can also be administered parenterally, in sterile liquid dosage forms. Other dosage forms are potentially possible such as patches or ointment or transdermal administration.

The lactoferrin products of the invention may also be formulated as a slow release implantation device for extended and sustained administration of the lactoferrin product. Examples of such sustained release formulations include composites of biocompatible polymers, such as poly(lactic acid), poly(lactic-co-glycolic acid), methylcellulose, hyaluronic acid, collagen, and the like. The structure, selection and use of degradable polymers in drug delivery vehicles have been reviewed in several publications, including, A. Domb *et al*., Polymers for Advanced Technologies 3:279-292 (1992). Additional guidance in selecting and using polymers in pharmaceutical formulations can be found in the text by M. Chasin and R. Langer (eds.), "Biodegradable Polymers as Drug Delivery Systems," Vol. 45 of "Drugs and the Pharmaceutical Sciences," M. Dekker, New York, 1990. Liposomes may also be used to provide for the sustained release of a lactoferrin product. Details concerning how to use and make liposomal formulations of drugs of interest can be found in, among other places, U.S. Pat. No 4,944,948; U.S. Pat. No. 5,008,050; U.S. Pat. No. 4,921,706; U.S. Pat. No. 4,927,637; U.S. Pat. No. 4,452,747; U.S. Pat. No. 4,016,100; U.S. Pat. No. 4,311,712; U.S. Pat. No. 4,370,349; U.S. Pat. No. 4,372,949; U.S. Pat. No. 4,529,561; U.S. Pat. No. 5,009,956; U.S. Pat. No. 4,725,442; U.S. Pat. No. 4,737,323; U.S. Pat. No. 4,920,016. Sustained release formulations are of particular interest when it is desirable to provide a high local concentration of a lactoferrin product.

Gelatin capsules or liquid-filled soft gelatin capsules may contain the active ingredient and powdered or liquid carriers, such as lactose, lecithin starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar-coated or film-coated to mask any unpleasant taste and to protect the tablet from the atmosphere, or enteric-coated for selective disintegration in the gastrointestinal tract. Liquid dosage forms for oral administration can contain coloring and/or flavoring to increase patient acceptance.

Suitable pharmaceutical carriers are further described in Remington's Pharmaceutical Sciences, 17th ed., Mack Publishing Company, Easton, PA (1990) a standard reference text in this field, which is incorporated herein by reference in its entirety.

In preferred embodiments, pharmaceutical compositions comprising lactoferrin products may be administered topically for the treatment of allergic skin disorders, as an inhalant for the treatment of pulmonary inflammatory diseases such as asthma, in the form of a nasal spray for rhinits and sinusitis, or in the form of injections for the treatment of arthritis. Besides the lactoferrin product, these compositions may comprise additional active components, including, but not limited to, hydrocortisone, retinoic acid, or conventional adjuncts used for current topical therapies. The preferred pharmaceutical dosage forms for the administration of the lactoferrin products of this invention can be illustrated as follows:

### Topical Formulation

In cases where treatment of inflammatory skin disorders is desired, the preferred route of administration will typically be topical. As lactoferrin is secreted, *e*.*g*., by sweat glands (Masson *et al*., 1966, *Clin. Chem. Axta* 14:735-739), lactoferrin products may generally be expected to enter through pores of the skin when administered topically. The availability of the active substance to its site of action will be further alleviated by the fact that inflammation is typically accompanied by disruption of the surface of the skin. Alternatively, substances which enhance the penetration through the skin may be added to the formulation.

Generally, for topical administration, the lactoferrin products may be formulated as a solution, gel, lotion, ointment, cream, suspension, paste, liniment, powder, tincture, aerosol, transdermal drug delivery system, and the like in a pharmaceutically acceptable form by methods well known in the art. Actual methods for preparing topical formulations are known or apparent to those skilled in the art, and are described in detail in Remington's Pharmaceutical Sciences, 17th ed., Mack Publishing Company, Easton, PA (1990); and Pharmaceutical Dosage Forms and Drug Delivery Systems, 6th ed., Williams & Wilkins (1995).

In order to enhance the percutaneous absorption of the active ingredients, a number of agents may be added in topical formulations, including, but not limited to, dimethylsulfoxide, dimethylacetamide, dimethylformamide, surfactants, azone, alcohol, acetone, propylene glycol and polyethylene glycol. In addition, physical methods may also be used to enhance transdermal penetration such as iontophoresis or sonophoresis.

The pharmaceutical compositions may be applied directly to the skin. Alternatively, they may be delivered by various transdermal drug delivery systems, such as patches.

### Formulations For Inhalants

In particular for the treatment pulmonary inflammatory diseases such as asthma and bronchitis, compositions comprising the lactoferrin product will typically be administered as an inhalant.

Generally, the lactoferrin product may be administered using a conventional inhaler. The lactoferrin product may be administered alone, or it may be co-administered with other inhalation aerosols, including, but not limited to, beta-adrenergic agonists, *e*.*g*., albuterol inhalation aerosols, salmeterol xinafoate inhalation aerosols, terbutaline sulfate inhalation aerosols; adrenocortical steroids, *e*.*g*., beclomethasone diproprionate inhalation aerosols; antiasthmatic, antiallergic, or mast cell stabilizers, *e*.*g*., cromolyn sodium inhalation aerosols; antiinflammatory aerosols, *e*.*g*., triamcinolone acetonide topical aerosol; anticholinergic agents, e.g., ipratropium bromide inhalation aerosols; or sympathomimetic substances, e.g., isoetharine mesylate inhalation aerosols, and metaproterenol sulfate inhalation aerosol. For further reference, additional teachings in these regards are provided in Pharmaceutical Dosage Forms and Drug Delivery Systems, by: Ansel *et al*., Williams & Wilkins, PA (1995).

### Formulations for sprays

In particular for the treatment of rhinitis, sinusitis and sunburn, compositions comprising the lactoferrin product will typically be administered as a spray.

Generally, the lactoferrin product may be administered using a conventional spray device such as those commercially available. The lactoferrin product may be administered alone, or it may be co-administered with other substances, including, but not limited to antihistamines, sympathomimetic agents and antibiotics. Moreover, the lactoferrin product may be administered in medicated sprays such as sodium chloride nasal solution (Salinex solution (Muro)) or Xylometazoline hydrochloride nasal solution (Sine-Off (SmithKline Beecham)) or functionally analogous sprays. For further reference, additional teachings in these regards are provided in Pharmaceutical Dosage Forms and Drug Delivery Systems, *supra*.

### Formulations For Injections

In particular for the treatment of arthritis and cellulitis, the compositions comprising a lactoferrin product may be administered by injection, e.g., locally into the diseased joint or cartilage, or systemically.

In general, water, oil, saline, aqueous dextrose (glucose), polysorbate and related sugar solutions and glycols such as propylene glycol or polyethylene glycols are suitable carriers for parenteral solutions. Solutions or emulsions for parenteral administration preferably contain about 5-15% polysorbate 80 or lecithin, suitable stabilizing agents, and if necessary, buffer substances. Antioxidizing agents, such as but not limited to, sodium bisulfite, sodium sulfite, or ascorbic acid, either alone or combined, are suitable stabilizing agents. Also used are citric acid and its salts and sodium EDTA. In addition, parenteral solutions can contain preservatives, including but not limited to, benzalkonium chloride, methyl- or propyl-paraben, and chlorobutanol.

Further suitable pharmaceutical carriers for parenteral administration are described in Remington's Pharmaceutical Sciences, *supra*.

### Indications

As discussed above, TNF-a plays important roles in the pathogenesis and/or progression of a variety of acute and chronic inflammatory disease processes. As a consequence, there has been considerable interest in designing synthetic inhibitors of TNF-α production that may be used therapeutically. The evidence cited herein indicates that lactoferrin products provide a means for regulating TNF-α production. The potential therapeutic applications of lactoferrin that derive from these data are many and include, but are not limited to, the treatment of the following discussed inflammatory disorders in a mammal, in particular a human in need.

### Contact Dermatitis

Contact dermatitis is an eczematous dermatitis caused by exposure to substances in the environment. Those substances act as irritants or allergens and may cause acute, subacute, or chronic eczematous inflammation. Irritant contact dermatitis, *i.e.*, irritation of the skin, is the most common form of contact dermatitis. Mild irritants may cause dryness, fissuring, and erythema, when exposure is continuous. For example, continuous exposure to moisture in areas such as the hand, the diaper area, *e.g*., infant diaper rash, or the skin around a colostomy may eventually cause eczematous inflammation. Strong chemicals may produce an immediate reaction. *See,* T.P. Habif, in: Clinical Dermatology, Mosby, Missouri (1996) and The Textbook of Medicine, *edts*: J.B. Wyngaarden and L.H. Smith, W., W.B. Saundres Company, Philadelphia (1985).

Allergic contact dermatitis, in contrast to irritant contact dermatitis, is a delayed hypersensitivity reaction that affects a limited number of individuals after one or a few exposures to a substance exhibiting antigenic activity on that particular individual.

Both irritant and allergic contact dermatitis are believed to be triggered by a local immune cascade involving *de novo* expression of TNF-α in keratinocytes as explained, *supra.*

***Current Treatment Methods And Their Drawbacks*****.** Acute contact dermatitis is currently treated with cold wet dressings, accompanied by the administration of steroids, *e*.*g*., hydrocortisone, prednisolone, methylprednisone, and, in severe cases, fluorinated corticosteriods, which are powerful anti-inflammatory agents at very low concentrations. Less potent than the fluorinated steroids, hydrocortisone, prednisolone and methylprednisone are used for less severe cases. Depending on the severity of the inflammation, the steroids are applied topically or systemically.

However, many adverse effects have been reported for steroids, especially in the case of the powerful fluorinated steroids. For example, epidermal and dermal atrophy can be a pronounced adverse effect and decreased collagen synthesis and reduced stromal support for blood vessels may lead to telangiectasia, purpura, and striae. A perioral dermatitis has also been reported with the fluorinated steroids, as well as aggravation of facial erythema, restricting absolutely the extended use of these compounds for the face. Further, possible elevated intraocular pressure warrants a strong proscription against the prolonged application of any topical steroids near the eyes.

The reality of systemic absorption of topical steroids presents an additional hazard. Lowering of plasma cortisol level is seen with as little as 20 per cent of the body under occlusion. The risk of rebound after discontinuing steroids in those skin diseases characterized by the phenomenon cannot be overlooked.

Overall, a need for low-toxicity treatment methods for contact dermatitis is apparent.

***Improved Treatment Methods Provided By The Present Invention*****.** The present invention provides novel pharmaceutical compositions comprising a lactoferrin product for the treatment of contact dermatitis. In a preferred embodiment, the compositions are formulated for topical application. Specifically, the lactoferrin product may be applied in the form of lotions, creams, ointments and the like, in concentrations of about 0.005% to about 5%, in preferred embodiments in concentrations of about 0.01% to about 0.5% active substance. Typically, the composition will comprise adjuncts conventionally used for topical administration. *See, supra*. In alternative embodiments, the compositions are formulated for intradermal injections. The compositions of the present invention directly interfere with the production of TNF-α by keratinocytes, thus preventing the exaggerated local immune response which is the underlying cause of irritant as well as allergic contact dermatitis. Since lactoferrin is known to be a non-toxic protein, the compositions provided will have non-detectable side effects.

### Pulmonary Inflammatory Diseases

Pulmonary Inflammatory Diseases such as allergic asthma and bronchitis are disorders characterized by increased responsiveness of the trachea and bronchi to various stimuli, i.e. allergens, resulting in widespread narrowing of the airways. For instance, asthma is an inflammatory reaction of the respiratory system in response to external stimuli. In severe cases, asthma may cause death through lack of oxygen supply and may afflict as many as five per cent of the population in the United States. In over half the cases, asthma is diagnosed between ages of two (2) and seventeen (17) years, and in this group it is the leading cause of disease and disability. *See,* The Textbook of Medicine, *edts:* J.B. Wyngaarden and L.H. Smith, W., W.B. Saundres Company, Philadelphia (1985).

***Current Treatment Methods And Their Drawbacks.*** Current treatment methods include the administration of sympathomimetic drugs, methylxanthines, and corticosteroids. Sympathomimetic drugs, including epinephrine and isoproterenol, have a beta-adrenergic effect. Their usefulness is limited by their actions on the heart, furthermore, tolerance develops after repeated use. Methylxanthines, which are believed to cause smooth muscle relaxation by their action on the cytoplasmic enzyme phosphodiesterase, in many cases cause anorexia, nausea, gastrointestinal upset, and central nervous system irritability. Corticosteroids are very effective in the treatment of asthma, however, they have severe side effects as discussed, *supra*. Thus, there is a need for new compositions and methods for the treatment of asthma which are effective while having low toxicity.

***Improved Compositions Provided By The Present Invention*****.** The present invention provides new compositions comprising a lactoferrin product and methods for the treatment of asthma. Typically, the compositions will be administered as an inhalant. Alternatively, compositions comprising the lactoferrin product may be administered systemically by injection or in the form of tablets, capsules, or in sustained release forms. Even at high dosage, the compositions of the invention will not have significant side effects, because lactoferrin is a natural, non-toxic protein.

The below examples explain the invention in more detail. The following preparations and examples are given to enable those skilled in the art to more clearly understand and to practice the present invention.

### EXAMPLES OF THE PREFERRED EMBODIMENTS

### Example 1

### Binding Of Lactoferrin To Receptors Located On TNF-α Producing Keratinocytes

The following example is an *in situ* ligand-binding assay showing the binding of ¹²⁵J-lactoferrin to keratinocytes in the epidermal layer of neonatal mouse skin.

***Experimental Procedure.*** Approximately 2 mm² back-skin samples from newborn wild type mouse were obtained and rinsed briefly twice with PBS at 37°C and incubated by duplicate at 37°C in 0.5 ml of PBS/0.1% bovine serum albumin contained:
(A) 1.0 x 10^{8 125}I-lactoferrin; and
(B) 1.0 x 10^{8 125}I-lactoferrin plus 50 folds excess unlabeled iron saturated recombinant lactoferrin.

Samples were washed six (6) times for ten (10) minutes each in cold PBS/0.1% bovine serum albumin and fixed in 4% paraformaldehyde for three (3) hours without shaking and then rinsed twice for ten (10) minutes in cold PBS. Immediately the samples were frozen. The samples were used to make 10 µm sections, and then the sections were dehydrated using ascending concentrations of ethanol. Sections were placed in NTB-2 emulsion and exposed for seven (7) and fourteen (14) days at 4°C. After development the sections were stained with hematoxylin and mounted with Permount.

***Binding Of Lactoferrin To Keratinocytes.*** As the *in situ* hybridization experiment reveals, lactoferrin accumulates at the membrane of keratinocytes. *See e.g.,* FIGURE 2. Specifically, FIGURE 2 shows the binding of lactoferrin to its receptors on TNF-α producing keratinocytes *in situ* on neonatal mouse skin. The top two panels show the bright and the dark field images obtained using only labelled lactoferrin (1.0 x 10⁸ ¹²⁵I-lactoferrin). The bottom two pabels show the bright and the dark field images when the binding assay was done in the presence of excess (50-fold excess) unlabelled lactoferrin. The binding of lactoferrin is concentrated in the epidermal layer and in the hair follicles, but is not observed in the normal dermal layer. These results indicate the binding of lactoferrin to its receptors on the surface of keratinocytes in the epidermis. Keratinocytes are known to produce TNF-α.

### EXAMPLE 2

### Influence Of Lactoferrin On DC Accumulation In Draining Lymph Nodes

The following example shows that lactoferrin inhibits the accumulation of dendritic cells in draining lymph nodes induced by oxazolone and IL-1β, while lactoferrin does not affect the accumulation of dendritic cells induced by administration of TNF-α.

***Experimental Procedure.*** Groups of mice (n=10) received 30µl intradermal injections into both ear pinnae of either 0.02% murine lactoferrin (LF) or 0.02% bovine serum albumin (BSA) each suspended in phosphate buffered saline (PBS; pH 7.2). Two (2) hours later, the mice received either a second intradermal injection at the same site of 50ng (30µl) of cytokine, *i.e.,* TNF-α or IL-1β, suspended in 0.1% BSA/PBS, or were exposed topically on the dorsum of both ears to 25µl of 0.5% oxazolone (Ox) dissolved in acetone:olive oil (4:1). Control mice were untreated. Draining (auricular) lymph nodes were removed at various times following exposure dependent upon kinetics of induced DC accumulation for each treatment, *i.e*., four (4) hours for TNF-α, seventeen (17) hours for IL-1β, and eighteen (18) hours for oxazolone, respectively. Subsequently, the number of dendritic cells per node was assessed as described previously. Cumberbatch and Kimber, 1992, *Immunology* 75 :257-263.

***Results*****.** As depicted in TABLE I and FIGURE 3A, treatment with oxazolone induced the accumulation of dendritic cells in draining lymph nodes (bar 4, in comparison to the control group depicted as bar 1). Intradermal administration of lactoferrin to the oxazolone-induced animals, however, resulted in a inhibition of accumulation of dendritic cells in the draining lymph nodes by 75% (Oxazolone/lactoferrin: 13,774 dendritic cells per node (above baselevel), oxazolone/+lactoferrin: 3,394 dendritic cells per node (above baselevel), *see,* TABLE I), indicating suppression of a local immune response in these animals by lactoferrin.

As further illustrated in TABLE I and FIGURE 3A, administration of TNF-α induced accumulation of dendritic cells in draining lymph nodes (*see,* FIGURE 3A, bar 2). As indicated by the results reflected by bar 3 of FIGURE 3A, lactoferrin does not have any effect on the TNF-α induced local immune response, *i*.*e*., administration of TNFα/-lactoferrin resulted in 4,753 dendritic cells per node (above baselevel), while administration of TNF- α/+lactoferrin resulted in 4,802 dendritic cells per node (above baselevel). These results indicate that lactoferrin acts upstream of TNF-α in the immune response cascade.

As depicted in TABLE I (B) and as further illustrated in FIGURE 3B, intradermal administration of IL-1β resulted in stimulation of Langerhans cell migration and dendritic cell accumulation in draining lymph nodes (FIGURE 3B, bar 1). The administration of lactoferrin resulted in an inhibition of the immune response. As indicated in FIGURE 3B, bar 2, intradermal injection of lactoferrin resulted in inhibition of dendritic cell accumulation by 56% (IL-1β/-lactoferrin: 7,569 dendritic cells per node (above baselevel), oxazolone/+lactoferrin: 3,342 dendritic cells per node (above base level), see, TABLE I).

These results indicate that:
(1) dendritic cell accumulation is dependent on increases in IL-1β levels, and
(2) IL-1β acts upstream of TNF-α, since a TNF-α induced immune response is not affected by lactoferrin.

Thus, IL-1β stimulates the *de novo* synthesis of TNF-α by keratinocytes. Furthermore, these data demonstrate that the inhibitory effect of lactoferrin on Langerhans cell migration and dendritic cell accumulation is due to the negative regulation of TNF-α production rather than via an influence on another aspect of Langerhans cell function. Thus, lactoferrin is able to inhibit the *de novo* synthesis of TNF-α.

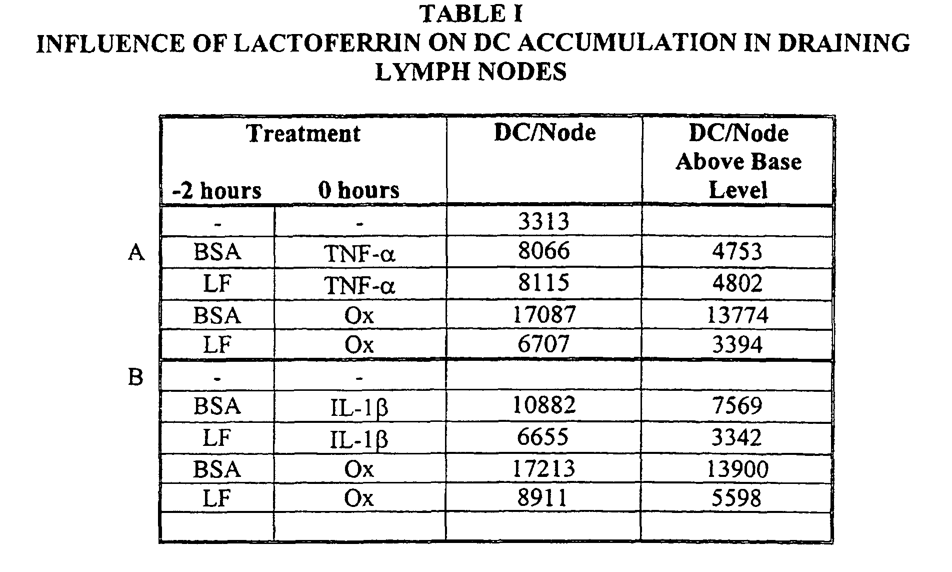

### EXAMPLE 3

### Influence Of Lactoferrin On TNF-α - And IL-1β-Induced Langerhans Cell Migration

The following example demonstrates that lactoferrin inhibits IL-1β -induced Langerhans cell migration, while it does not affect migration of Langerhans cells which is induced by TNF-α.

***Experimental Procedure.*** Groups of mice (n=3) received 30µl intradermal injections in both ear pinnae of either 0.005% murine lactoferrin (LF) or 0.005% bovine serum albumin (BSA) each suspended in phosphate buffered saline (PBS; pH 7.2). Two hours later mice received a second intradermal injection at the same site of 50 ng (30µl) of cytokine, *i*.*e*., TNF-α or IL-1β, respectively, suspended in 0.1 % BSA/PBS. Control mice were untreated. Ears were removed 30 mins following TNF-α treatment or 17 hours following IL-1β treatment and epidermal sheets prepared as described previously. Cumberbatch *et al*., 1994, *Immunology* 81:395-401. The frequency of Langerhans cells (LC) was measured by indirect immunofluorescence. Results are expressed as the mean number of cells/mm³ (±SE) derived from examination of 10 fields/sample for each of 4 samples. The statistical significance of differences between 5 experimental groups was calculated using the Student's t-test. p.<0.005.

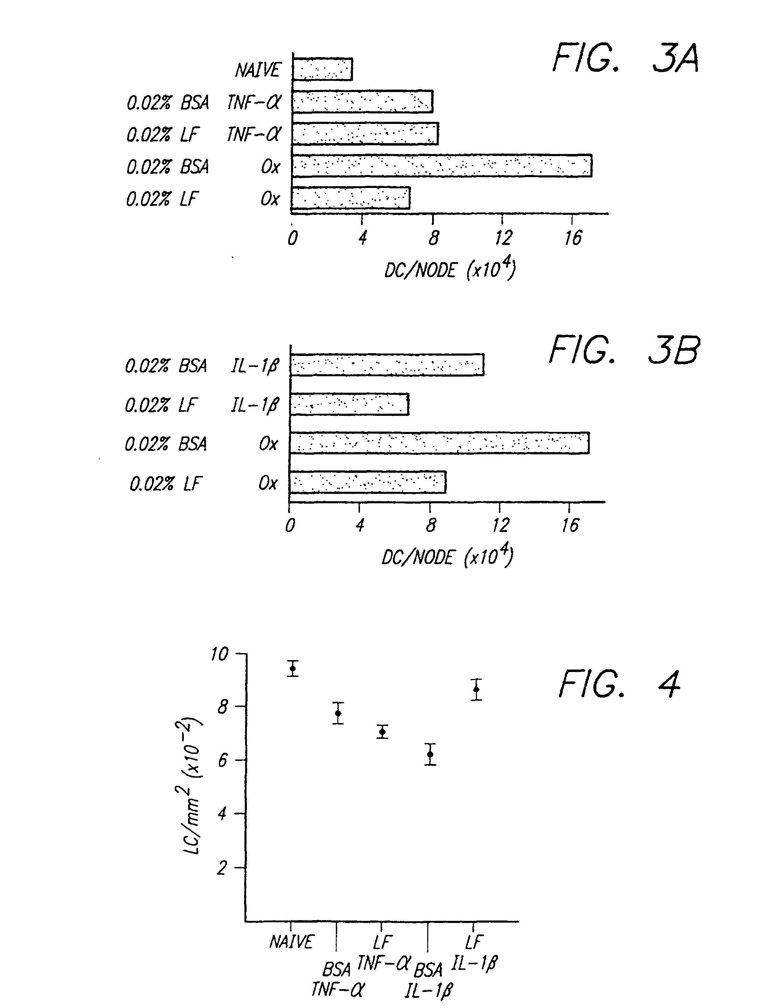

***Results.*** As depicted in TABLE II and FIGURE 4, intradermal administration of IL-1β resulted in stimulation of Langerhans cell migration, which was inhibited by lactoferrin. Also administration of TNF-α resulted in migration of Langerhans cells, however, TNF-α induced migration was not inhibited by lactoferrin.

10 These results confirm that IL-1β acts upstream of TNF-α in the local immune response cascade, and that lactoferrin acts downstream of IL-1β, but upstream of TNF-α.

**TABLE II**

| **INFLUENCE OF LACTOFERRIN ON TNF-A AND IL-1β--INDUCED LANGERHANS CELL MIGRATION.** | | |
|---|---|---|
| **Treatment** | | **Langerhans Cells per mm**^{**2**} |
| **-2 hours** | **0 hours** | |
| - | - | 938.9±22.2 |
| BSA | TNF-α | 766.5±36.9 |
| LF | TNF-α | 701.2±21.2 |
| BSA | IL-1β | 615.6±16.1 |
| LF | IL-1β | 858.9±28.9 |

### EXAMPLE 4

### Influence of lactoferrin on the accumulation of dendritic cells in draining lymph nodes

The following example shows that lactoferrin, when administered topically, inhibits the accumulation of dendritic cells in draining lymph nodes induced by oxazolone.

**Experimental Procedure.** Groups of mice (n=6) received topically a 0.02% solution of lactoferrin dissolved in lubriderm lotion or 0.02% bovine serum albumin (BSA) dissolved similarily on the dorsum of both ears. Later, the mice were exposed topically at the same sites to 25ul of 0.5% oxazolone (Ox) dissolved in acetone:olive oil (4:1). Control mice were untreated. Draining lymph nodes were removed 18 hours following exposure and the number of dendritic cells per node was assessed as described previously. Cumberbatch *et al*., 1992, Immunology 75:257-263.

**Results.** As depicted in Figure 5, results from two independent experiments (panels A and B) indicate that treatment with oxazolone induced accumulation of dendritic cells in draining lymph nodes of mice pretreated with 0.02% BSA (see bar 2 panels A and B versus bar 1 control). Topical administration of mouse lactoferrin resulted in a strong inhibition of accumulation of dendritic cells in the lymph nodes in response to oxazolone in both experiments (see bar 3, panels A and B).

The results indicate that topically administered lactoferrin is highly effective at inhibiting dendritic cell accumulation in lymph nodes in response to an allergen and hence allergen-induced cutaneous inflammation.

Figure 5 shows the inhibitory effect of lactoferrin on oxazolone induced dendritic cell accumulation in draining lymph nodes when lactoferrin is applied topically to the skin surface.

## Claims

1. Use of a lactoferrin product for the production of a medicament for treating an allergen-induced inflammatory disorder in a mammal, wherein the allergen-induced inflammatory disorder is asthma.

2. Use of a lactoferrin product for the production of a medicament for treating an allergen-induced inflammatory disorder in a mammal, wherein the allergen-induced inflammatory disorder is contact dermatitis and wherein said medicament consists of a lactoferrin product and a pharmaceutical carrier.

3. The use of claim 1 or 2, wherein the mammal is a human.

4. The use according to any one of claims 1 to 3, wherein the lactoferrin product is a naturally occurring lactoferrin.

5. The use according to any one of claims 1 to 3, wherein the lactoferrin product is a recombinantly produced lactoferrin.

6. The use according to any one of claims 1 to 4, wherein the lactoferrin product is a biologically active fragment of lactoferrin.

7. The use according to any one of claims 1 to 6, wherein the lactoferrin product is administered as an inhalant.

## Patentansprüche

1. Verwendung eines Lactoferrinprodukts für die Herstellung eines Arzneimittels zur Behandlung einer Allergen-induzierten entzündlichen Erkrankung in einem Säuger, wobei die Allergen-induzierte entzündliche Erkrankung Asthma ist.

2. Verwendung eines Lactoferrinprodukts für die Herstellung eines Arzneimittels zur Behandlung einer Allergen-induzierten entzündlichen Erkrankung in einem Säuger, wobei die Allergen-induzierte entzündliche Erkrankung Kontaktdermatitis ist und wobei das Arzneimittel aus einem Lactoferrinprodukt und einem pharmazeutischen Träger besteht.

3. Verwendung gemäß Anspruch 1 oder 2, wobei der Säuger ein Mensch ist.

4. Verwendung gemäß einem der Ansprüche 1 bis 3, wobei das Lactoferrinprodukt ein natürlicherweise vorkommendes Lactoferrin ist.

5. Verwendung gemäß einem der Ansprüche 1 bis 3, wobei das Lactoferrinprodukt ein rekombinant erzeugtes Lactoferrin ist.

6. Verwendung gemäß einem der Ansprüche 1 bis 4, wobei das Lactoferrinprodukt ein biologisch aktives Fragment von Lactoferrin ist.

7. Verwendung gemäß einem der Ansprüche 1 bis 6, wobei das Lactoferrinprodukt als Inhalat verabreicht wird.

## Revendications

1. Utilisation d'un produit lactoferrine pour la production d'un médicament pour traiter un désordre inflammatoire induit par un allergène chez un mammifère, dans laquelle le désordre inflammatoire induit par un allergène est l'asthme.

2. Utilisation d'un produit lactoferrine pour la production d'un médicament pour traiter un désordre inflammatoire induit par un allergène chez un mammifère, dans laquelle le désordre inflammatoire induit par un allergène est l'eczéma de contact et dans laquelle ledit médicament est constitué d'un produit lactoferrine et d'un support pharmaceutique.

3. Utilisation selon la revendication 1 ou 2, dans laquelle le mammifère est un humain.

4. Utilisation selon l'une quelconque des revendications 1 à 3, dans laquelle le produit lactoferrine est une lactoferrine existant à l'état naturel.

5. Utilisation selon l'une quelconque des revendications 1 à 3, dans laquelle le produit lactoferrine est une lactoferrine produite de manière recombinante.

6. Utilisation selon l'une quelconque des revendications 1 à 4, dans laquelle le produit lactoferrine est un fragment biologiquement actif de lactoferrine.

7. Utilisation selon l'une quelconque des revendications 1 à 6, dans laquelle le produit lactoferrine est administré comme un produit inhalé.
